Europäisches Patentamt

European Patent Office

Office européen des brevets

(11) Publication number: **0 241 252 B1**

# EUROPEAN PATENT SPECIFICATION

(45) Date of publication of patent specification: **25.03.92**  (51) Int. Cl.⁵: **A61L  27/00**, A61F 2/08

(21) Application number: **87302979.7**

(22) Date of filing: **06.04.87**

(54) **Artificial, absorbable ligament prosthesis.**

(30) Priority: **07.04.86 US 848549**

(43) Date of publication of application:
**14.10.87 Bulletin  87/42**

(45) Publication of the grant of the patent:
**25.03.92 Bulletin  92/13**

(84) Designated Contracting States:
**AT BE CH DE ES FR GB IT LI NL SE**

(56) References cited:
**FR-A- 2 156 513**
**GB-A- 2 159 846**

(73) Proprietor: **JOHNSON & JOHNSON ORTHO-
PAEDICS INC.
501 George Street
New Brunswick New Jersey 08903(US)**

(72) Inventor: **Benicewicz, Brian C.
5800 Eubank N.E. 2631
Albuquerque New Mexico(US)**
Inventor: **Oser, Zale
46 Shadey Lane
Bound Brook New Jersey(US)**
Inventor: **Clemow, Alastair J.T.
1085 Cherry Hill Road R.D. 1, Box 245
Princeton New Jersey(US)**
Inventor: **Shalaby, Shalaby Wabba
238-A Longview Road Mountainville, R.D. 2
Lebanon, NJ(US)**

(74) Representative: **Jones, Alan John et al
CARPMAELS & RANSFORD 43 Bloomsbury
Square
London, WC1A 2RA(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to artificial tendons and ligaments, and more specifically, to ligament and tendon prosthesis which are absorbable in the human body.

Artificial tendon and ligament prosthesis have been discussed in the literature for many years. By artificial tendons and ligaments it is meant replacements for human and animal ligaments which are not homografts or xenografts, but are synthetic materials which are implanted in the human or animal body as a replacement for a ligament or tendon. The original tendon or ligament prosthesis were considered to be permanent replacements for a natural ligament or tendon and were made from materials which were compatible in the body and which were intended to completely replace the tendon or ligament. Examples of these materials are disclosed in the following patents:

U.S. Patent No. 3,176,316 discloses a tendon made with a solid, flexible shaft surrounded by a finely braided woven or intertwined synthetic plastic material such as polytetrafluoroethylene. The fibrous material extended beyond the shaft to allow for attachment of the prosthesis to body tissue.

U.S. Patent No. 3,513,484 discloses a woven polyester tape covered with silicone rubber and with stainless steel loops at the ends to attach the prosthesis to the bone or muscle.

U.S. Patent No. 3,545,008 discloses a tendon prosthesis made from polyester mesh or similar material and coated with a silicone rubber in essentially the same manner as U.S. Patent 3,513,484. This tendon has a flap at each end to envelop the severed ends of the tendon.

U.S. Patent No. 3,797,047 discloses a tendon prosthesis which is composed of a silicone elastomer tube which has a polyester fiber inserted in the tube. The polyester fibrous portion of the ligament is free to move within the tube to prevent adhesions.

U.S. Patent No. 3.805,300 discloses a tendon or ligament prosthesis which is made of strands of flexible filaments coated with a biocompatable material. Portions of the fibrous material have openings or fenestrations to allow for the ingrowth of tissue. In use, the natural tendon can be split and woven through the fenestrations in the prosthesis to secure the prosthesis in place.

U.S. Patent No. 3,953,896 discloses a ligament prosthesis which is made from an ultrahigh, molecular weight polyethylene and which has a particular attachment mechanism to attach the prosthesis in place. This ligament prosthesis is particularly designed for use as a cruciate ligament in the human knee.

U.S. Patent 4,034,763 discloses a ligament forming suture made from a loosely woven or an expanded plastic material which should have sufficient porosity to allow tissue to penetrate the suture.

U.S. Patent No. 3,992,725 discloses a prosthesis which is made from a highly porous material such as polytetrafluoroethylene and carbon fibers with substantial void spaces in between which allow for tissue ingrowth.

All of the above prosthesis were generally considered to be permanent in that they would replace the function of the natural tendon or ligament. The permanent-type prosthesis mentioned above have not met with much success because as they are a mechanical device; it was known that the device most likely would eventually fail and would require other surgical procedures to correct the failure of this mechanical device.

More recently, it has been determined that it is possible for the human and animal body to reconstruct damaged tissues and ligaments if there was an implant of a porous structure which would provide a scaffolding for new tissue to be deposited. A prosthesis of this type is disclosed in the following patents:

U.K. Patent No. 1,602,834 discloses a carbon fiber prosthesis which is composed of twisted carbon fiber filaments which are relatively open and allow tissue to grow along and between the fibers.

U.S. Patents Nos. 4,329,743 and 4,411,027 disclose a similar prosthesis in which the carbon fibers are covered with a bioabsorbable polymer such as polylactide. The purpose of the coating was to attempt to prevent the breakage and subsequent migration of the carbon fibers which had been found to break into fragments which migrate throughout the joint, and potentially throughout the body.

The carbon fiber prosthesis have not met with wide success because of the problem of the brittle carbon fibers breaking with time and finding their way throughout the body of the patient. The coated carbon fiber prosthesis also have a tendency to break and migrate after the coating has lost its mechanical integrity or has been absorbed by the body.

U.S. Patent 3,297,033 discloses an absorbable suture material made from polyglycolide.

U.S. Patents 3,636,956; 3,797,499 and 4,137,921 disclose absorbable sutures made from homopolymers of lactide and copolymers of lactide and glycolide.

U.S. Patents 4,539,981 and 4,550,449 disclose an absorbable bone fixation device made from a high molecular weight polylactide polymer. The process of forming the polymer employs a monomer to catalyst mole ratio from 1000 to 300,000.

Brief Description of the Drawings

Figure 1 is a graph comparing the in vivo tensile strength retention characteristics of a prosthesis of the present invention with prosthesis made from commercially available absorbable suture materials.

Figure 2 is a graph illustrating the in vivo tensile strength retention of the ligament prosthesis of the present invention.

Summary of the Invention

Applicants have developed a tendon and ligament prosthesis free of the undesirable features which led to the rejection and/or functional failure of the prosthesis disclosed in the prior art. The present tendon and ligament prosthesis are made from an absorbable polymer and is used as a transient ligament or tendon prosthesis which dissociates in vivo to the biologically compatible lactic and/or glycolic acid. By transient, it is meant that upon implantation, the prosthesis will provide a scaffold to induce natural tissue to form along the filaments of the prosthesis. After the natural tissue has grown along the filaments and formed a new ligament or tendon, the filaments will be absorbed, and within some time period will no longer be present at the implant site or anywhere in the body. This prosthesis avoids the problems with the carbon fiber-type filaments as the absorbable polymer will not fragment and migrate through the body, and even if there was some fragmentation and migration of the ligament or tendon prosthesis of the present invention, the particles would be absorbed.

Absorbable filamentary materials, particularly absorbable sutures, have been known for some years. These sutures are generally polyglycolide as disclosed in the Schmitt, U.S. Patent No. 3,297,033, or polylactide such as that disclosed in the Schneider patent, No. 3,636,956, or copolymers of 90% glycolide and 10% lactide such as disclosed in U.S. Patent No. 3,836,297. Other copolymers of lactide and glycolide have also been proposed as absorbable sutures. However, all of these prior art materials are not suitable for the prosthesis of the present invention for one or more reasons. The sutures made from polymers which are predominately glycolide would fail to meet the in vivo mechanical strength, (i.e., tensile strength) requirements of the scaffold to allow sufficient tissue ingrowth in the critical time period as they are unacceptably fast absorbing for use as a ligament or tendon prosthesis. The prior art sutures made from polymers which are predominately lactide could not be made into a filament which would have the requisite strength and dimensions to be used as a ligament or tendon prosthesis. This is particularly true of the filaments disclosed in the Schneider U.S. Patent 3,636,956, which could not be melt-spun at a high enough temperature to produce fibers of the small diameter necessary to manufacture a ligament prosthesis with the high surface area and the porosity for optimum tissue ingrowth.

Applicants have now discovered that it is possible to manufacture an artificial tendon and ligament prosthesis by employing a lactide polymer or a lactide copolymer which contains up to 10 mole percent glycolide, and which is end-capped with a monofunctional alcohol having from 6-14 carbon atoms. The preferred monofunctional alcohols are the higher boiling point normal alcohols having 10 to 14 carbon atoms. The particular alcohol most preferred is dodecanol. A high molecular weight polymer or high degree of polymerization polymer made, using a trace amount of an organo metallic catalyst i.e., $1 \times 10^{-4}$ to $2 \times 10^{-5}$ mole percent and preferably $5 \times 10^{-5}$ to $2 \times 10^{-5}$ mole percent, in accordance with the teachings of the present invention can be melt-spun at temperatures in excess of 210°C, and thereby form extremely fine filaments which have the requisite initial strength, and which have the requisite breaking strength retention (BSR) to be used as a ligament or tendon prosthesis. The melt spinning of a high molecular weight polymer into strong fine fibers can only be achieved with thermostable polymers at the temperatures required to obtain optimum melt viscosity to produce uniform fine denier fibers. Filaments made according to the present invention can be plied, slightly twisted or braided into a size to provide the strength necessary for any particular tendon or ligament application. The twist or braid will provide a relatively open structure which is geometrically capable of allowing natural tissue to be deposited along the filaments and develop natural tendon or ligamentous tissue. After this tissue is formed, the filamentary material will eventually be absorbed by the body and will not present any problems relating to the migration of material through the body. The prosthesis of the present invention may be attached to a bone cutting needle or other type needle. The prosthesis may also be coated with an absorbable coating for ease of handling. The absorbable coating should absorb at a faster rate than the polymer from which the filaments of the prosthesis are formed. A preferred absorbable coating is the 65/35 L(-)lactide/glycolide copolymer disclosed in Mattei, U.S. Patent No. 4,027,676. The higher fatty acids or esters of higher fatty acids disclosed in the coating of the Mattei patent are not employed in the coating used in the present prosthesis. The coating is applied from a solvent to provide a coating level of about 1% by weight or less, preferably about 0.2% by weight, based on the weight of the uncoated prosthesis.

Detailed Description of the Invention

Generally, the filaments of the present invention are made in a fashion similar to that taught in prior art for the manufacture of poly(2-hydroxyacid) materials. A combination of low catalyst concentration and chain end capping in the present process produces a high lactide polymer suitable for melt spinning to high tenacity fibers. The polymers used in the prosthesis of the present invention are made from L(-)lactide and up to 10 mole percent glycolide. The polymers are polymerized in an inert atmosphere with an organo-metallic catalyst at a temperature of from about 110 to about 180°C. The monofunctional alcohol, preferably dodecanol, used an end cap, is added to the reaction mass before the polymerization is initiated. The dodecanol is added in an amount to give a monomer to dodecanol mole ratio of from 600 to 1400 to 1. The polymerization proceeds for a period of from about 2 1/2 hours to 72 hours to produce a polymer having an inherent viscosity; measured at a concentration of 0.1 g/dl in chloroform at 25°C of from 1.4 to 2.7 dl/g. The preferred inherent viscosity of the polymer is between 1.9 and 2.3 dl/g. The polymer is removed from the reaction vessel, chilled and ground into small particles. The ground polymer is devolatilized to remove unreacted monomer and stored under vacuum until use. In effect, the major differences in the present process versus that taught in the prior art polylactide polymerization processes is the selection of a particular monomer to catalyst ratio, and the use of a monofunctional alcohol having from 6 to 14 carbon atoms to act as the end cap on the polymer chains. The catalyst is present in the polymerization mass in a monomer to catalyst mole ratio of from 10,000 to 50,000 to 1 and may be as high as 120,000 to 1. The monomer to catalyst ratio is preferably from 20,000 to 40,000 to 1. The preferred organo metallic catalyst is stannous octoate. The use of the monofunctional alcohols as the end caps provides a melt stability to the polymer when the polymer is melt-spun. Prior art polylactide polymers were not capable of being melt-spun at temperatures above about 210°C. At spinning temperatures of 210°C and below, fine filaments cannot be spun. By contrast, the polymer of the present invention can be melt-spun at temperatures of from 215° to 240°C and above. Because the present high molecular weight polymers can be spun at these temperatures, extremely fine strong filaments can be made, and these filaments are particularly desirable in a ligament or tendon prosthesis. The fine filaments of the present invention have a diameter which provides a high surface area comparable to the dimensions of the cells in the body where the ligaments would be implanted. It is believed that the high surface area and small diameter fibers are desirable to maximize tissue ingrowth. The diameter of the filaments of the present invention is between about 3 and 15 μm and preferably between 5 and 12 μm.

The present polymers can be spun at temperatures of 240°C without excessive degradation of the polymer. The polymers are spun through a die having 20 to about 60 openings, preferably between about 28 and 40 openings. As the filaments are collected in a yarn, they are coated with a spin lubricant such as mineral oil. The yarn is drawn over rolls heated to a temperature of about 80° to 120°C. The yarn is then plied and twisted or braided into a prosthesis of the desired diameter. The total diameter of the prosthesis should be between 20 and 80 mils (508 and 2032 μm) depending on the natural ligament to be replaced. Generally, a diameter of 40 to 50 mils (1016 to 1270 μm) is adequate for most replacements.

The filaments of the present invention are generally formed into a lightly or moderately twisted structure, or can be formed into a braid in the same manner as sutures. A loosely-braided structure will provide sufficient areas for tissue ingrowth. However, the preferred structure is a lightly-twisted tow of filaments having 1 to 15 and preferably 2 to 8 twists per inch (2.54 cm).

The braided or twisted yarn may be annealed by heating the yarn while the yarn is under tension. The yarn is heated to a temperature of from 80°C to 120°C in a nitrogen atmosphere for a period of from 6 to 24 hours.

As previously indicated, the twisted or braided yarn may be coated with an absorbable coating to improve handling. The yarn may be mechanically pliablized and then is cut to the desired length for a prosthesis and the cut ends may be coated or tipped with tipping agent to prevent brooming and to allow easy attachment of a needle. The tipping agent may be any of those agents used in tipping sutures.

The finished prosthesis, with or without an attached needle, is sterilized preferably with ethylene oxide and packaged in a moisture impermeable package.

In order to function as a ligament prosthesis, the present materials will maintain their strength for extended periods of time. The breaking strength retention of the present materials are such that the implanted devices maintain at least 30% of the original strength after a period of at least three months and preferably six months when implanted in animals. The most preferred materials will maintain at least 50% of the original strength for at least six months. This strength retention requirement cannot be satisfied by any of the commercially available absorbable sutures.

As the inherent strength of the present prosthesis is gradually diminished because of absorption in the body, the natural tissue is being deposited along the fibers to provide functional strength to support the new ligament. The rate at which the natural tissue is deposited about the absorbable fiber scaffold must be in concert with the rate of strength loss of the fiber. If the fiber scaffold looses its strength before adequate natural tissue has been deposited, the prosthesis will fail.

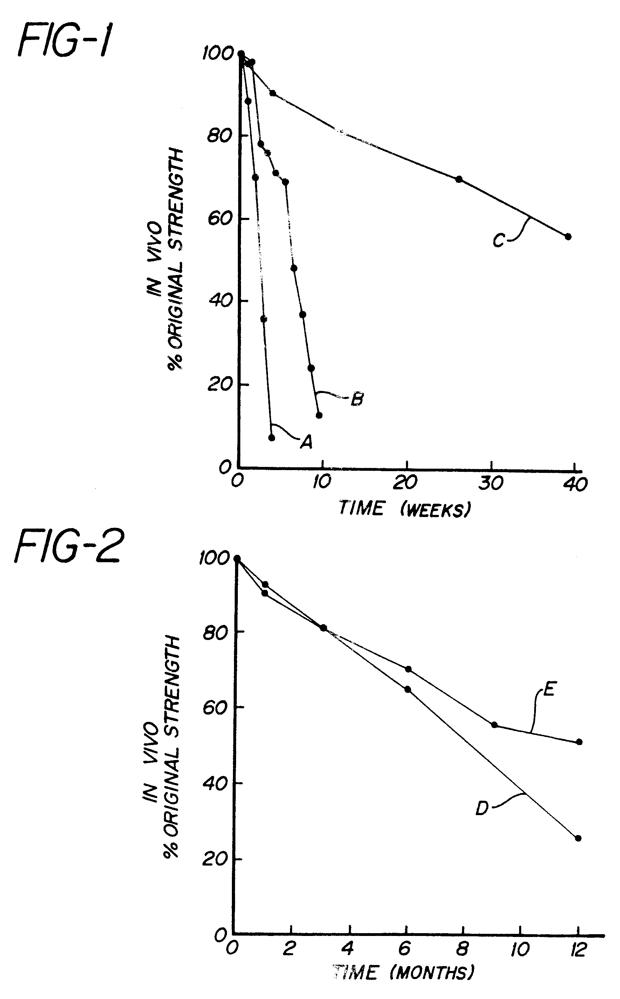

The tensile strength retention characteristics of the polymer material of the present invention is compared to commercial absorbable suture materials in Fig. 1. Curve A in Fig. 1 is a 10/90 poly(L-lactide-co-glycolide) suture material disclosed in U.S. patent 3,839,297. Curve B is a poly-p-dioxanone suture material of the type disclosed in U.S. patent 4,052,988. Curve C is the material of the present invention. The breaking strength retention data for curves A and B is from information published in Handbook of Fiber Science and Technology: Volume III, High Technology Fibers, Part A, Edited by Levin and Preston, Marcel Dekker, Inc. 1985. Fig. 1 illustrates the improved breaking strength retention characteristics of the material of the present invention.

Fig. 2 is a graph of the breaking strength retention characteristic of the material of the present invention for time periods of up to 12 months. Curve D is the copolymer of Example 1 and Curve E is the homopolymer of Example 2. Fig. 2 illustrates that the present materials will retain more than 30% of their original strength for time periods of three months and longer, providing adequate strength while new tissue is deposited.

The polymers used in the present invention can be polymerized by a solid state or a melt polymerization process. In the solid state process, the polymerization continues after the polymer has become solid. In the melt polymerization process, the polymerization is terminated while the polymer is a melt at the polymerization temperature.

If desired, the absorbable filaments of the present invention may be combined with non absorbable fibers, such as high tenacity polyester or gel-spun ultra high strength polyethylene fibers to form composite tendon or ligament prosthesis. The combined fibers might be used in applications where extremely high strength would be desirable for longer time periods than those described for totally absorbable prosthesis. The prosthesis of the present invention may be employed in combination with and to reinforce autologous tissue in a standard autologous tissue graft.

EXAMPLE 1

PREPARATION, PROCESSING AND TESTING OF POLY(L)LACTIDE-CO-GLYCOLIDE 95/5

Two identical runs are made under the given below conditions to provide a sufficient quantity of polymer for extrusion and testing. Two 1-liter round bottom flasks are equipped with overhead mechanical stirrers and flame dried under vacuum to remove moisture from the interior surfaces of the vessels. When cool, the vacuum is released by introducing dry nitrogen gas. To each of the flasks is added, in a dry nitrogen glovebox, a mixture of 479.7 grams (3.328 moles) of pure L(-)lactide and 20.3 grams (0.175 moles) of pure glycolide. This mixture is 5 mole percent glycolide and 95 mole percent L(-)lactide.

To each of these reaction mixtures is added 0.50 ml of a 0.33 molar catalyst solution (stannous octoate in toluene) containing $1.65 \times 10^{-4}$ moles of stannous octoate using a dry glass syringe. The molar ratio of monomer to catalyst is 21,230:1. Then, 1.06 ml ($4.66 \times 10^{-3}$ moles) of warm 1-dodecanol is added to each flask. The molar ratio of monomer to 1-dodecanol is 750:1. The reaction vessels are closed and high vacuum is applied to remove the toluene. After a minimum of a few hours, the vacuum is released to dry nitrogen gas and the contents of the flasks are kept under dry nitrogen atmosphere by use of gas bubblers which apply a slight positive pressure of nitrogen to the flasks. The vessels and their contents are lowered into silicone oil baths, preheated to a temperature of 120°C. Stirring is started as the monomers melt and continued until the viscosity of the reactants becomes too high to stir. The stirrers are lifted out of the reaction mixture and the reaction is continued for three days at 120°C. The vessels are cooled and the polymer masses are removed from the vessel. The polymer masses are chilled in liquid nitrogen, divided into pieces, and ground in a Cumberland Mill. The ground polymers are placed in trays in a vacuum oven to dry and subsequently devolatilized at 110°C for 18 hours under vacuum. After cooling, the polymers are removed from the oven and stored under vacuum. The inherent viscosity of this copolymer (at a concentration of 0.1 g/dl in chloroform at 25°C) is 1.96 dl/g.

The polymer batch described above is extruded under dry nitrogen using a screw extruder to produce a 28-strand multifilament yarn. The polymer is extruded at a temperature of about 240°C. The multifilament yarn is coated with a spinning lubricant and oriented 4x by stretching over a godet heated at 80°C. The average tensile strength of the multifilament (60 denier -6.66 tex) yarns is 3.4 grams per denier and displays an elongation to break of 61%.

The 28-strand yarn (60 denier -6.66 tex) is placed on braider bobbins on a 12-carrier machine with a 7-ply core. The braid is made with 51 picks per inch (2.54 cm) and hot stretched 25% at 215°F (102°C).

The properties of the braid was:

| | |
|---|---|
| Average tensile load at break | 7.05 ± 0.23 lb (3.2 ± 0.1 kg) |
| Average diameter | 15.4 ± 0.3 mils (391 ± 8 $\mu$m) |

The braid is annealed under tension at 100°C for 24 hours in a nitrogen atmosphere.

The annealed braid is scoured to remove the spin lubricant and any exterior contaminants by placing the braid in a scouring tank filled with hexane for between fifteen and thirty minutes. The braid is air dried for at least 30 minutes after scouring.

The properties of the annealed braid were:

| | |
|---|---|
| Average tensile load at break | 7.89 ± 0.10 lb (3.6 ± .04 kg) |
| Average diameter | 14.4 ± 0.25 mils (366 ± 6 $\mu$m) |

The in vitro breaking strength retention (BSR) and absorption were determined by the following procedure: The braided yarn is kept in a phosphate buffer at pH 7.27 at 50°C for the required period of time. At the end of each period sufficient samples are removed from the bath, rinsed with water and dried to constant weight at 25°C under reduced pressure. The change in molecular weight of the dry samples and a control (not subjected to the phosphate buffer) is measured in terms of inherent viscosity (I.V.) in $CHCl_3$. Similarly the breaking strength retention is determined through measurement of the breaking strength of the dry samples using an Instron tensiometer. The in vitro absorption of braided samples treated as shown above is determined in terms of the percent mass loss.

In vitro changes in molecular weight (measured in terms of inherent viscosity; I.V.) and loss of breaking strength in pH 7.27 buffer at 50°C are:

| Time, wks. | I.V. dl/q | % BSR |
|---|---|---|
| 0 | 1.47 | (100) |
| 4 | 1.11 | 84.9 |
| 8 | 0.83 | 57.0 |
| 12 | 0.38 | 14.7 |
| 16 | 0.36 | 0 |

The in vitro absorption data, in pH 7.27 buffer (0.1 grams braid in 100 ml buffer) at 50°C is as follows:

| Time, wks. | % Weight remaining |
|---|---|
| 0 | 100 |
| 4 | 99.8 |
| 12 | 95.3 |
| 26 | 58.3 |
| 39 | 20.5 |
| 52 | 2.1 |
| 65 | 1.2 |

The filaments were also tested to determine breaking strength retention in vivo by the following procedure: The breaking strength retention in vivo of a fiber is determined by implanting two strands of the fiber in the dorsal subcutis of each of a number of Long-Evans rats. The number of rats used is a function of the number of implantation periods, employing 4 rats per period giving a total of eight (8) examples for each of the periods. The periods of in vivo residence are 7, 31, 92, 184, 276 or 365 days. The ratio of the mean value of 8 determinations of the breaking strength (determined with an Instron tensile tester employing the following settings: a gauge length of 1 inch (2.54 cm), a chart speed of 1 inch/minute (2.54 cm/min), and a crosshead speed of 1 inch/minute -2.54 cm/min) at each period to the mean value (of 8 determinations) obtained for the fiber prior to implantation constitutes its breaking strength retention for that period.

The results were as follows:

| Time, Days | % BSR |
|---|---|
| 0 | (100) |
| 7 | 101 |
| 31 | 93 |
| 92 | 81 |
| 184 | 65 |
| 368 | 26 |

The in vivo results shown above are illustrated by curve D in Fig. 2.

EXAMPLE 2

PREPARATION, PROCESSING & TESTING OF POLY(L-)LACTIDE

Two identical runs are made under the following conditions to provide a sufficient quantity of polymer for extrusion and testing. Two 1-liter round bottom flasks are equipped with overhead mechanical stirrers and flame dried under vacuum to remove moisture from the interior surfaces of the vessels. When cool, the vacuum is released by the introduction of dry nitrogen gas. To each of the flasks is added, in a dry nitrogen glovebox, 500.0 grams (3.469 moles) of pure L(-)lactide.

To each of these reaction mixtures, 0.35 ml of a 0.33 molar catalyst solution (stannous octoate in toluene) containing $1.155 \times 10^{-4}$ moles of catalyst is added using a dry glass syringe. The molar ratio of monomer to catalyst is 30,000:1. Then, 1.06 ml ($4.66 \times 10^{-3}$ moles) of warm 1-dodecanol is added to each flask. The molar ratio of monomer to 1-dodecanol is about 750:1. The reaction vessels are closed and a high vacuum is applied to remove the toluene. After a minimum of a few hours, the vacuum is released to dry nitrogen gas and the contents of the flasks are kept under nitrogen atmosphere by use of gas bubblers which apply a slight positive pressure of nitrogen to the flasks. The vessels and their contents are lowered into silicone oil baths, preheated to a temperature of 120°C. Stirring is started as the monomers melt and continued until the viscosity of the reactants becomes too high to stir. The stirrers are lifted out of the reaction mixture and the reaction is continued for 3 days at 120°C. The vessels are cooled and the polymer masses are removed from the vessel. They are chilled in liquid nitrogen, cut into pieces, and ground in a Cumberland Mill. The ground polymers are placed in a vacuum oven to dry and subsequently devolatilized at 110°C for 18 hours under vacuum. After cooling, the polymers are removed from the oven and stored under vacuum. The inherent viscosity of these polymers (at a concentration of 0.1 g/dl in chloroform at 25°C) are 2.28 and 2.32 dl/g.

The polymer described above were combined and extruded under dry nitrogen using a screw extruder to produce a 28-strand multifilament. The combined polymers were extruded at a temperature of about 240°C. The multifilament is oriented 8.33x by stretching over a godet heated at 120°C. The average tensile strength of the multifilament (32 denier 3.55 tex) yarns is 4.4 grams per denier (39 CN/tex) and displays an elongation to break of 33%.

The 28 strand yarn (32 denier 3.55 tex) is placed on braider bobbins on a 12-carrier machine with a 7-ply core. The braid is made with 51 picks per inch (2.54 cm) and hot stretched 24% at 215°F (102°C).

Properties of the Braid were:

| Average Tensile Load at Break | 6.10 ± 0.29 lb. (2.8 ± 0.13 kg) |
|---|---|
| Average Diameter | 10.8 ± 0.38 mils (274 ± 10 μm) |

The inherent viscosity of the braid at a concentration of 0.1 g/dl in chloroform at 25°C is 1.56 dl/g. The braid is annealed under tension at 100°C for 24 hours in a nitrogen atmosphere.

The annealed Braid Properties were:

7

| Average Tensile Load at Break | 5.17 ± 2.24 lb. (2.3 ± 1.0 kg) |
| Average Diameter | 8.7 ± 0.2 mils (221 ± 5 $\mu$m) |

The annealed braid was scoured as in Example I. The breaking strength retention and the absorption were determined as in Example I. The results are as follows:

In vitro BSR, and changes in molecular weight and Breaking Strength in pH 7.27 buffer at 50°C were:

| Time, Wks | I.V. dl/q | % BSR |
| --- | --- | --- |
| 0 | 1.56 | 100 |
| 1 | 1.47 | 95.7 |
| 3 | 1.40 | 91.3 |
| 4 | 1.24 | 88.4 |

| Time, Wks | I.V. dl/g | % BSR |
| --- | --- | --- |
| 6 | 1.19 | 83.0 |
| 8 | 1.17 | 76.8 |
| 12 | 0.94 | 56.1 |
| 16 | 0.80 | 28.6 |
| 26 | 0.29 | 0 |

The in vitro absorption at 50°C, pH 7.27 buffer were:

| Time, Wks | % Weight Remaining |
| --- | --- |
| 0 | 100 |
| 4 | 100 |
| 12 | 99.5 |
| 26 | 95.9 |
| 39 | 91.9 |
| 52 | 58.5 |
| 65 | 23.3 |

The filaments were also tested in vivo for BSR and absorption as in Example I and the results are as follows:

| Time, Days | % BSR |
| --- | --- |
| 0 | 100 |
| 7 | 98 |
| 31 | 90 |
| 92 | 82 |
| 184 | 70 |
| 276 | 56 |
| 365 | 52 |

The in vivo results shown above are illustrated by curve C in Fig. 1 and curve E in Fig. 2.

EXAMPLE 3

PREPARATION & PROCESSING OF POLY(L-)LACTIDE

Five runs are made under the following conditions to provide a sufficient quantity of polymer for extrusion and testing. The 1-liter round bottom flasks are equipped with magnetic stirrers and flame dried under vacuum to remove moisture from the interior surfaces of the vessels. When cool, the vacuum is released to dry nitrogen gas. To each of the flasks is added, in a dry nitrogen glovebox, 700.0 grams (4.857 moles) of pure L(-)lactide.

To each of these reaction mixtures 0.49 ml of a 0.33 molar catalyst solution (stannous octoate in toluene) containing .62 x $10^{-4}$ moles catalyst was added using a dry glass syringe. The molar ratio of monomer to catalyst is 30,000:1. Then, 1.47 ml (6.47 x $10^{-3}$ moles) of warm 1-dodecanol is added to each flask. The molar ratio of monomer to 1-dodecanol is 750:1. The reaction vessels are closed and a high vacuum is applied to remove the toluene. After a minimum of a few hours, the vacuum is released to dry nitrogen gas and the contents of the flasks are kept under nitrogen atmosphere by use of gas bubblers which apply a slight positive pressure of nitrogen to the flasks. The vessels and their contents are lowered into silicone oil baths, preheated to a temperature to 110°C. Stirring is started as the monomers melt and continued until the viscosity of the reactants becomes too high and stops the magnetic stirring bar. The reaction is continued for 3 days at 110°C. The vessels are cooled and the polymer masses are removed from the vessel. The polymer masses are chilled in liquid nitrogen, cut into pieces, and the polymers are ground in a Cumberland Mill. The inherent viscosity of the polymers at a concentration of 0.1 g/dl in chloroform at 25°C ranges from 2.19 to 2.31 dl/g. The ground polymers are combined and devolatilized and dried in a tumble dryer at 120°C for 18 hours under vacuum. After cooling, the polymers are removed from the dryer and stored under vacuum.

The polymer batch described above is extruded under dry nitrogen using a screw extruder to produce a 28-strand multifilament which is oriented 9x by stretching over a godet heated at 130°C. The average tensile strength of the multifilament (30 denier -3.3 tex) yarns is 4.8 grams per denier and displays an average elongation to break of 35%.

## EXAMPLE 4

### PREPARATION & PROCESSING OF POLY(L-)LACTIDE

Three identical runs are made under the following conditions to provide a sufficient quantity of polymer for extrusion and testing. The 1-liter round bottom flasks are equipped with magnetic stirrers and flame dried under vacuum to remove moisture from the interior surfaces of the vessels. When cool, the vacuum is released to dry nitrogen gas. To each of the flasks is added, in a dry nitrogen glovebox, 700.0 grams (4.857 moles) of pure L(-)lactide.

To each of these reaction mixtures 0.49 ml of a 0.33 molar catalyst solution (stannous octate in toluene) containing 1.62 x $10^{-4}$ moles catalyst is added using a dry glass syringe. The molar ratio of monomer to catalyst is 30,000:1. Then, 1.47 ml (6.47 x $10^{-3}$ moles) of warm 1-dodecanol is added to each flask. The molar ratio of monomer to 1-dodecanol is 750:1. The reaction vessels are closed and a high vacuum is applied to remove the toluene. After a minimum of a few hours, the vacuum is released to dry nitrogen gas and the contents of the flasks are kept under nitrogen atmosphere by use of gas bubblers which apply a slight positive pressure of nitrogen to the flasks. The vessels and their contents are lowered into silicone oil baths, preheated to a temperature of 110°C. Stirring is started as the monomers melt and continued until the viscosity of the reactants becomes too high and stops the magnetic stirring bar. The reaction is continued for 3 days at 120°C. The vessels are cooled and the polymer masses are removed from the vessel. They are chilled in liquid nitrogen, cut into pieces, the stirring bar removed and the polymers are ground in a Cumberland Mill. The inherent viscosity of the polymers (at a concentration of 0.1 g/dl in chloroform at 25°C ranges from 1.48 to 1.90 dl/g. The ground polymers are combined and devolatilized and dried in a tumble dryer at 120°C for 18 hours under vacuum. After cooling, the polymers are removed from the dryer and stored under vacuum.

The polymer batch described above is extruded under dry nitrogen using a screw extruder to produce a 28-strand multifilament. The polymer is extruded at a temperature of 240°C. The multifilament is oriented 10x by stretching over a godet heated at 130°C. The average tensile strength of the multifilament (28 denier -3 tex) yarns is 4.7 grams per denier (41 CN/tex) and displays an average elongation to break of 36%.

## EXAMPLE 5

### PREPARATION & PROCESSING OF POLY(L-)LACTIDE

A 2-liter round bottom flask is equipped with an overhead mechanical stirrer and flame dried under vacuum to remove moisture from the interior surfaces of the vessel. When cool, the vacuum is released to dry nitrogen gas. To the flask is added, in a dry nitrogen glovebox, 1900.0 grams (13.183 moles) of pure L(-)lactide.

To this reaction 1.33 ml of a 0.33 molar catalyst solution (stannous octoate in toluene) containing $4.39 \times 10^{-4}$ moles catalyst is added using a dry glass syringe. The molar ratio of monomer to catalyst is 30,000:1. Then, 3.33 ml ($1.47 \times 10^{-2}$ moles) of warm 1-dodecanol is added to each flask. The molar ratio of monomer to 1-dodecanol is 900:1. The reaction vessel is closed and a high vacuum is applied to remove the toluene. After a minimum of a few hours, the vacuum is released to dry nitrogen gas and the contents of the flask is kept under nitrogen atmosphere by use of a gas bubbler which applies a slight positive pressure of nitrogen to the flask. The vessel and its contents is lowered into a silicone bath, preheated to a temperature of 110°C. Stirring is started as the monomer melts and is continued until the viscosity of the reactants becomes too high to stir. The stirrer is lifted out of the reaction mixture and the reaction is continued for 3 days at 110°C. The vessel is cooled and the polymer mass is removed from the vessel. The polymer is chilled in liquid nitrogen, cut into pieces, and ground in a Cumberland Mill. The ground polymer is placed in trays in a vacuum oven and devolatilized at 110°C for 18 hours under vacuum. After cooling, the polymer is removed from the oven and stored under vacuum. The inherent viscosity of this polymer (at a concentration of 0.1 g/dl in chloroform at 25°C) is 2.24 dl/g.

The polymer described above is extruded under dry nitrogen using a screw extruder to produce a 28-strand multifilament. The polymer is extruded at a temperature of 240°C. The multifilament is oriented 10x by stretching over a godet heated at 135°C. The average tensile strength of the multifilament (28 denier -3 tex) yarns is 5.0 grams per denier (44 CN/tex) and the multifilament displays an average elongation to break of 39%.

## EXAMPLE 6

### TWISTING OF POLY(L-)LACTIDE YARNS

The yarns produced in Examples 3, 4 and 5 are 10 plied onto 16 carrier bobbins and twisted at 6 turns per inch (2.36 turns per cm). The twisted bundle has an approximate diameter of 32 mils (813 $\mu$m).

The Unannealed Properties of the twisted bundle were:

| | |
|---|---|
| Average Tensile Load at Break: | 45 lbs. (20 kg) |
| Average Elongation at Break: | 36% |

The twisted bundle is wound onto annealing racks under slight tension and annealed in an oven at 95°C for 15 hours in a nitrogen atmosphere. The twisted bundle has an approximate diameter of 34 mils after annealing.

The annealed Properties of the twisted bundle were:

| | |
|---|---|
| Average Tensile Load at Break: | 39 lbs. (17.7 kg) |
| Average Elongation at Break: | 37% |

The twisted bundles of annealed fibers are scoured as in Example I.

The twisted bundles are fitted with straight needles, packaged in foil packs, and sterilized by ethylene oxide.

## EXAMPLE 7

### PREPARATION, PROCESSING & TESTING OF POLY(L-)LACTIDE

A thoroughly dried, mechanically stirred 2.5 gallon (9.5 ℓ) stainless steel reactor is charged with 5000 grams (34.70 moles) of pure L(-)lactide. To this reactor is added 6.47 grams ($3.47 \times 10^{-2}$ moles) of 1-dodecanol. The molar ratio of monomer to the alcohol is 1000:1. To this reactor 4.2 ml of a 0.33 molar catalyst solution (stannous octoate in toluene) containing $1.389 \times 10^{-3}$ moles of catalyst is added using a dry glass syringe. The molar ratio of monomer to catalyst is 25,000:1. The reactor is purged and vented with nitrogen. The reactor is heated to 190°C. The stirring is started when the monomer is melted. The temperature is maintained at 190°C for 3 hours after which the polymer is discharged from the reactor and ground in a Cumberland Mill. The ground polymer is dried and devolatilized under the following conditions:

vacuum pressure of 200 micron (μm)/18 hours at 25°C, followed by

vacuum pressure of 200 microns (μm) for 24 hours at 110°C followed by

vacuum pressure of 200 microns (μm) for 4 hours at 25°C

The inherent viscosity of this polymer (at a concentration of 0.1 g/dl in chloroform at 25°C) was 1.94 dl/g.

The polymer described above was extruded under dry nitrogen at a temperature of 240°C using a screw extruder to produce a 28-strand multifilament. The multifilament was oriented 10x by stretching over a godet heated at 120°C. The average tensile strength of the multifilament (28 denier -3 tex) was 5.7 grams per denier (50 CN/tex) and displays an elongation to break at 43%.

The 28 strand yarn (28 denier 3 tex) was 2 plied and placed on braider bobbins on a 12-carrier machine with a 7-ply core. The braid was made with 46 picks per inch and hot stretched 24% at 215°F (102°C).

The braid Properties were:

| | |
|---|---|
| Average Tensile Load at Break | 8.51 ± 0.46 lb. (3.9 ± 0.21 kg) |
| Average Diameter | 14.5 ± 0.4 mils (368 ± 10 μm) |

The braid was annealed under tension at 100°C for 24 hours in a nitrogen atmosphere.

The annealed Braid Properties were :

| | |
|---|---|
| Average Tensile Load at Break | 7.70 ± 0.10 lb. (3.5 ± 0.05 kg) |
| Average Diameter | 13.5 ± 0.4 mils (343 ± 10 μm) |

The inherent viscosity of the braid at 0.1 percent concentration in chloroform at 25°C is 1.60 dl/g.

The annealed braid is scoured as in Example I.

The in vitro BSR, changes in molecular weight and breaking strength in pH 7.27 buffer at 50°C:

| Time, Wks | I.V. dl/g | % BSR |
|---|---|---|
| 0 | 1.60 | 100 |
| 1 | 1.59 | 91.8 |
| 3 | 1.54 | 88.6 |
| 4 | 1.46 | 88.3 |
| 6 | 1.35 | 89.2 |
| 10 | 1.14 | 74.7 |
| 12 | 1.03 | 64.8 |
| 14 | 0.91 | 52.4 |
| 16 | 0.80 | 46.2 |
| 18 | 0.73 | 36.9 |
| 20 | ---- | 34.3 |

EXAMPLE 8

The Achilles tendon of white New Zealand rabbits was used as the model for neo-tendon formation, where a unilateral one centimeter defect is repaired using a scaffold implant.

A total of three materials were examined:

1. Twisted Carbon fiber (3000 fibers/tow)

2. Braided Size (Polylactide) fibers from Example 6

3. Twisted Size (Polylactide) fibers from Example 6

The different construction of the implants was as follows:

| Fiber | Construct. | # Fibers | Max Load |
|---|---|---|---|
| Carbon | Twisted | 6000 | 425N |
| PLA | Braided | 5376 | 200N |
| PLA | Twisted | 4480 | 173N |

The form of PLA fiber used in this study retains 85% of its strength after 12 weeks in vivo and 65% after 26 weeks as shown in Example 6. Two animals underwent a sham operation in which a similar tendon defect was made but no implant inserted. These shams were tested immediately post-operatively to obtain baseline data.

The tendons were examined post-operatively at 0, 4, 8, 12 and 26 weeks. Four animals were used for each implant at each time period. At sacrifice, the regrown tendons were mechanically tested to failure at 1"/sec. (2.54 cm/sec) with the contralateral limb serving as a control. The parameters measured included the stiffness and yield load. At the conclusion of the test, the central section of the regrown tendon was dissected out and either fixed in formalin for histological sectioning or frozen for future collagen studies.

A summary of the mechanical test data is presented below with the mean and standard error from the mean being given as shown in the following tables:

| Yield Load (% of Contralateral) | | | | |
|---|---|---|---|---|
| Time Wks. | Carbon | PLA Braid | PLA TWIST | Sham |
| 0 | 32.3±3.4 | 39.7± 3.3 | 42.4±1.6 | 28.2±4.0 |
| 4 | 55.7±5.1 | 67.3±10.6 | 95.5±6.5 | |
| 8 | 85.5±6.8 | 80.8± 5.5 | 87.2±4.7 | |
| 12 | 82.5±9.0 | 89.5± 7.4 | 89.5±7.4 | |
| 26 | 74.7±10.9 | 98.7±3.9 | 103.7±2.9 | |

| Stiffness (% of Contralateral) | | | | |
|---|---|---|---|---|
| Time Wks. | Carbon | PLA Brand | PLA TWIST | Sham |
| 0 | 46.2±3.0 | 42.3±4.5 | 47.4±2.8 | 39.0 ± 1.4 |
| 4 | 67.8±5.9 | 61.4±6.5 | 76.3±6.4 | |
| 8 | 73.5±5.4 | 77.6±3.9 | 69.4±4.6 | |
| 12 | 82.4±3.2 | 81.1±7.3 | 73.6±1.4 | |
| 26 | 82.7±3.2 | 82.9±7.1 | 83.6±4.9 | |

The yield load data indicates the strengths of the tendons increased with time to approximately 85% of normal within 8 weeks. A significantly higher strength was observed for the twisted PLA fibers than for the carbon fibers at the 4 week period and the 26 week period. At 26 weeks, the PLA implants were found to result in approximately 100% of normal strength while the carbon fiber implants had decreased in strength.

EXAMPLE 9

PREPARATION AND PROCESSING OF POLY(L(-)LACTIDE-CO-GLYCOLIDE) 95/5 MOLAR RATIO

A melt polymerization, is carried out in a throughly dried, mechanically stirred two gallon (7.6 ℓ) stainless steel oil heated reactor. The ingredients to be charged into the reactor are 5 kilograms (34.69 moles) of pure L(-) lactide, 212.0 grams (1.826 moles) of pure glycolide, 5.68 grams ($3.048 \times 10^{-2}$ moles) of 1-dodecanol and 2.77 milliliters of a 0.33 molar catalyst solution containing stannous octate in toluene ($9.14 \times 10^{-4}$ moles). The molar ratio of lactide to glycolide is 95 to 5. The molar ratio of monomer to catalyst is 40,000 to 1 and the molar ratio of monomer to alcohol is 1200 to 1. The polymerization is conducted as follows:

One-half of the L(-)lactide is placed in the unit, followed by a mixture of all the glycolide and dodecanol. The last half of the L(-)lactide is added and the stannous octate solution is carefully added using a dry glass syringe and long needle. The stirrer is put in reverse.

12

The unit is evacuated below 1 mm of mercury pressure for twenty minutes and then restored to atmospheric pressure with dry nitrogen. The evacuation procedure is repeated. The circulating oil in the reactor jacket is heated to 130°C. When the temperature of the reaction mass reaches 120°C the rotation of the stirrer is put in forward at 15 RPM and the oil temperature controller is set to rise an average of 1° per minute until the oil reaches 185°C. The oil temperature is maintained at 180°C for 90 minutes. The oil temperature is raised to 190°C, the stirrer speed is dropped to 8 to 10 RPM and the nitrogen pressure is raised to maintain a steady exit of the polymer melt from the bottom port. The melted polymer is cooled to a solid, ground, dried and devolatilized under less than 200 $\mu$ pressure. The properties of the polymer are given below:

```
Inherent viscosity,                        2.04 dl/g
Concentration of 0.1 g/dl at 25°, in hexafluoriso-
propyl alcohol
Melt index, 235°C                          0.394 g/10 min
Volatiles                                  1.37%
Tg                                         61°C
Tm                                         162°C
```

The polymer of the Example was suitable for melt spinning into fine strong filaments which would be useful in making the ligament of the present invention. A tendon prosthesis made from this polymer had the following characteristics:

| | |
|---|---|
| Yarn Size | 57 denier/40 filaments (6.4 tex/40 filament) |
| Denier per filament | 1.43 (0.16 tex per filament) |
| Filament diameters | Average 9.17 $\mu$m |
| Filament diameter range | 8 to 12 $\mu$m |
| Tenacity of filament | 5.1 g/d (grams per denier) -(45 CN/tex) |
| Collation | 13 ply of above yarn |
| Twist bobbins | 19 |
| Total number of fiber bundles in the tendon | 247 |
| Total denier of tendon | 14,079 (1.6 tex) |
| Diameter of tendon | 51.99 mils ± 2.72 (1320 ± 69 $\mu$m) (S.D.) |
| Straight tensile Breaking Strength | 129.47 lbs (59 kg) |
| Straight tensile (Psi) | 60,883 psi (4.2 x $10^5$ kPa) |
| Elongation % | 33.4 |
| Yield stress | 44.25 lbs (20 kg) |
| Yield stress (Psi) | 20,482 psi (1.4 x $10^5$ kPa) |

## Claims

1. An absorbable ligament and tendon prosthesis, capable of maintaining at least 30% of its initial tensile strength for a period of at least 3 months after implantation in an animal body, comprising a plurality of fine filaments arranged in an open structure to allow tissue ingrowth between individual filaments, said filaments having a diameter of from 3 to 15 $\mu$m and comprising a melt spinnable polymer stable at temperatures above 210°C and containing from 90 to 100 mole % L(-)lactide and 0 to 10 mole % glycolide, said polymer being end-capped with a monofunctional alcohol having from 6 to 14 carbon atoms.

2. The prosthesis of claim 1, wherein the monofunctional alcohol is dodecanol.

3. The prosthesis of claim 1, or claim 2, in which the filaments have a diameter of from 5 to 12 $\mu$m.

EP 0 241 252 B1

**4.** The prosthesis of any one of claims 1 to 3, in which the prosthesis has a total diameter of from 0.76 to 1.52 mm (30 to 60 mils).

**5.** The prosthesis of any one of claims 1 to 4, in which the filaments are plied into a twisted yarn having from 1 to 15 twists per 2.54 cm (inch) of length.

**6.** The prosthesis of claim 5, in which the number of twists per 2.54 cm (inch) is between 2 and 8.

**7.** The prosthesis of any one of claims 1 to 6, in which the total number of filaments in the prosthesis is between 3,000 and 10,000.

**8.** The prosthesis of claim 7, in which the total number of filaments in the prosthesis is between 4,000 and 8,000.

**9.** A process of forming a ligament and tendon prosthesis comprising polymerizing a reaction mass containing 100 to 90 mole % L(-)lactide and 0 to 10 mole % glycolide in an inert atmosphere in the presence of a catalyst, said catalyst being present in a monomer to catalyst mole ratio of from 10,000 to 50,000 to 1, said reaction mass containing a monofunctional alcohol containing from 6 to 14 carbon atoms in a monomer to alcohol mole ratio of from 600 to 1400 to 1, spinning the polymer into fibers having a diameter of between 3 and 15$\mu$m, and plying the fibers into a porous structure having a diameter of between 0.051 and 0.20 cm (20 and 80 mils).

**10.** The process 9, in which the spinning temperature of the polymer is between 215°C and 240°C.

**11.** The process of claim 9 or claim 10, in which the catalyst is an organometallic catalyst.

**12.** The process of claim 11, in which the catalyst is stannous octoate.

**13.** The process of any one of claims 9 to 12, in which the monofunctional alcohol is dodecanol.

**14.** The process of any one of claims 9 to 13 which is used to produce the prosthesis of any one of claims 1 to 8.

**Revendications**

**1.** Une prothèse de ligament et de tendon, susceptible d'être absorbée et de maintenir au moins 30 % de leur résistance initiale à la traction pendant une période d'au moins 3 mois après implantation dans le corps d'un animal, comportant une pluralité de fins filaments agencés en structure ouverte afin de permettre un développement interne du tissu entre les filaments individuels, lesdits filaments présentant un diamètre de 3 à 15 mm et comportant un polymère susceptible d'être filé à l'état fondu, stable à des températures au-dessus de 210°C et renfermant de 90 à 100 moles % de L(-)lactide et 0 à 10 moles % de glycolide, ledit polymère étant coiffé aux extrémités par un alcool monofonctionnel présentant de 6 à 14 atomes de carbone.

**2.** La prothèse de la revendication 1, dans laquelle l'alcool monofonctionnel est le dodécanol.

**3.** La prothèse de la revendication 1 ou de la revendication 2, dans laquelle les filaments ont un diamètre de 5 à 12 $\mu$m.

**4.** La prothèse de l'une quelconque des revendications 1 à 3, dans laquelle la prothèse présente un diamètre total de 0,76 à 1,52 $\mu$m (30 à 60 mils).

**5.** La prothèse de l'une quelconque des revendications 1 à 4, dans laquelle les filaments sont pliés en un filé tordu présentant 1 à 15 torsions par 2,54 cm (pouce) de longueur.

**6.** La prothèse de la revendication 5, dans laquelle le nombre de torsions par 2,54 cm (pouce) se situe entre 2 et 8.

14

**7.** La prothèse de l'une quelconque des revendications 1 à 6, dans laquelle le nombre total de filaments dans la prothèse se situe entre 3000 et 10 000.

**8.** La prothèse de la revendication 7, dans laquelle le nombre total de filaments dans la prothèse se situe entre 4000 et 8000.

**9.** Un procédé pour réaliser une prothèse de ligament et de tendon, dans lequel on polymérise une masse réactionnelle renfermant de 100 à 90 moles % de L(-)lactide et de 0 à 10 moles % de glycolide dans une atmosphère inerte en présence d'un catalyseur, ledit catalyseur étant présent dans un rapport molaire entre le monomère et le catalyseur de 10 000 à 50 000 à 1, ladite masse réactionnelle renfermant un alcool monofonctionnel renfermant de 6 à 14 atomes de carbone selon un rapport molaire du monomère à l'alcool de 600 à 1400 à 1, on file le polymère en fibres présentant un diamètre entre 3 et 15 $\mu$m, et on ordonne les fibres en une structure poreuse présentant un diamètre entre 0,051 et 0,20 cm (20 et 80 mils).

**10.** Le procédé de la revendication 9, dans lequel la température de filage du polymère se situe entre 215°C et 240°C.

**11.** Le procédé de la revendication 9 ou de la revendication 10, dans lequel le catalyseur est un catalyseur organométallique.

**12.** Le procédé de la revendication 11, dans lequel le catalyseur est l'octoate stanneux.

**13.** Le procédé de l'une quelconque des revendications 9 à 12, dans lequel l'alcool monofonctionnel est le dodécanol.

**14.** Le procédé de l'une quelconque des revendications 9 à 13, que l'on met en oeuvre pour fabriquer la prothèse de l'une quelconque des revendications 1 à 8.

**Patentansprüche**

**1.** Absorbierbare Bänder- und Sehnenprothese, welche befähigt ist, wenigstens 30 % ihrer anfänglichen Zugfestigkeit während einer Zeitspanne von wenigstens drei Monaten nach ihrer Implantation in einem Tierkörper beizubehalten, und welche eine Mehrzahl von feinen Fäden umfaßt, die in einer offenen Struktur angeordnet sind, um das Hineinwachsen von Gewebe zwischen den einzelnen Fäden zu ermöglichen, wobei diese Fäden einen Durchmesser von 3 bis 15 $\mu$m aufweisen und ein aus der Schmelze spinnbares Polymer enthalten, welches bei Temperaturen über 210°C stabil ist und welches 90 bis 100 Mol-% L-(-)-Lactid und 0 bis 10 Mol-% Glycolid enthält, wobei dieses Polymer mit einem monofunktionellen Alkohol mit 6 bis 14 Kohlenstoffatomen endverkappt ist.

**2.** Prothese nach Anspruch 1, worin der monofunktionelle Alkohol Dodecanol ist.

**3.** Prothese nach Anspruch 1 oder 2, wobei die Fäden einen Durchmesser von 5 bis 12 $\mu$m haben.

**4.** Prothese nach einem der Ansprüche 1 bis 3, wobei die Prothese einen Gesamtdurchmesser von 0,76 bis 1,52 mm (30 bis 60 Mil) hat.

**5.** Prothese nach einem der Ansprüche 1 bis 4, wobei die Fäden zu einem verzwirnten Garn gefacht sind, welches 1 bis 15 Zwirnungen je 2,54 cm (1 Zoll) seiner Länge aufweist.

**6.** Prothese nach Anspruch 5, wobei die Anzahl der Zwirnungen je 2,54 cm (1 Zoll) zwischen 2 und 8 beträgt.

**7.** Prothese nach einem der Ansprüche 1 bis 6, wobei die Gesamtzahl der Fäden in der Prothese zwischen 3.000 und 10.000 ausmacht.

**8.** Prothese nach Anspruch 7, wobei die Gesamtzahl der Fäden in der Prothese zwischen 4.000 und 8.000 ausmacht.

9. Verfahren zum Ausbilden einer Bänder- und Sehnenprothese, welches das Polymerisieren einer Reaktionsmasse, welche 100 bis 90 Mol-% L-(-)-Lactid und 0 bis 10 Mol-% Glycolid enthält, in einer inerten Atmosphäre, in Gegenwart eines Katalysators, wobei dieser Katalysator in einem Molverhältnis von Monomeren zu Katalysator von 10.000 bis 50.000 zu 1 vorliegt, und wobei diese Reaktionmasse einen monofunktionellen Alkohol mit einem Gehalt von 6 bis 14 Kohlenstoffatomen in einem Molverhältnis von Monomeren zu Alkohol von 600 bis 1400 zu 1 enthält; das Verspinnen des Polymers zu Fäden mit einem Durchmesser zwischen 3 und 15 $\mu$m, und das Fachen der Fäden zu einer porösen Struktur mit einem Durchmesser zwischen 0,051 und 0,20 cm (20 und 80 Mil) umfaßt.

10. Verfahren nach Anspruch 9, wobei die Temperatur des Verspinnens des Polymers zwischen 215° C und 240° C liegt.

11. Verfahren nach Anspruch 9 oder 10, wobei der Katalysator ein metallorganischer Katalysator ist.

12. Verfahren nach Anspruch 11, wobei der Katalysator Zinn(II)-octoat ist.

13. Verfahren nach einem der Ansprüche 9 bis 12, wobei der monofunktionelle Alkohol Dodecanol ist.

14. Verfahren nach einem der Ansprüche 9 bis 13, welches zur Erzeugung einer Prothese nach einem der Ansprüche 1 bis 8 angewendet wird.

FIG-1

FIG-2